# EUROPÄISCHE PATENTSCHRIFT

(11) **EP 1 100 345 B1**
(45) Veröffentlichungstag und Bekanntmachung des Hinweises auf die Patenterteilung: **29.10.2003**
(21) Anmeldenummer: 99948667.3
(22) Anmeldetag: 26.07.1999
(51) Int. Cl.: A23L 1/10, A23L 1/185, A23L 3/3472

(54) **MITTEL ZUR STABILISIERUNG VON LEBENSMITTELN UND KOSMETISCHEN MITTELN SOWIE VERFAHREN ZU DESSEN HERSTELLUNG**
AGENT FOR STABILIZING FOODSTUFFS AND COSMETIC AGENTS, AND A METHOD FOR THE PRODUCTION THEREOF
AGENT POUR STABILISER DES PRODUITS ALIMENTAIRES ET DES PRODUITS COSMETIQUES, ET SON PROCEDE DE PRODUCTION

(30) Priorität: 01.08.1998 DE 19834764
(43) Veröffentlichungstag der Anmeldung: 23.05.2001
(73) Patentinhaber: Universität Hannover, 30167 Hannover (DE)
(72) Erfinder: EL SAHARTY, Yasser, Shaker, Ibrahim, El Maadi Cairo 11432 (EG); KRINGS, Ulrich, D-31628 Landesbergen (DE); BERGER, Ralf, Günther, D-30455 Hannover (DE)
(74) Vertreter: Eisenführ, Speiser & Partner
(86) Internationale Anmeldenummer: DE9902258
(87) Internationale Veröffentlichungsnummer: WO00007464

(56) Entgegenhaltungen:
- EP-A- 0 513 354
- EP-A- 0 659 347
- WO-A-90/01271
- GB-A- 2 263 856
- US-A- 4 022 921
- KAJIMOTO, G. ET AL.: "Antioxidant effects of barley aqeous extract on the oxidative deterioration of oil." NIPPON EIYO SHOKURYO GAKKAISHI - JOURNAL OF JAPANESE SOCIETY OF NUTRITION AND FOOD SCIENCE, Bd. 44, Nr. 11, 1997, Seiten 788-794, XP000866197 TOKYO., JP ISSN: 0287-3516
- PATENT ABSTRACTS OF JAPAN vol. 014, no. 126 (C-0699) & JP 02 003495 A (OKUNO SEIYAKU KOGYO)

## Beschreibung

Die Erfindung betrifft ein Mittel zur Stabilisierung von Lebensmitteln und kosmetischen Mitteln sowie ein Verfahren zu dessen Herstellung.

Insbesondere lipidreiche Lebensmittel und kosmetische Mittel können infolge von Lipid-Peroxidationsprozessen (Autoxidation) ranzig werden. Dieses Ranzigwerden von Lebensmitteln (häufig erkennbar durch ihren kratzigen, unangenehmen Geschmack) und kosmetischen Mitteln führt zu ihrer Unbrauchbarkeit.

Es ist bereits bekannt, Lebensmitteln und kosmetischen Mitteln natürliche oder synthetische Antioxidantien zuzusetzen, um ihre Autoxidation zu hemmen. Typische Antioxidantien sind zum Beispiel *tert*-Butylmethoxyphenol (*tert*-Butylhydroxyanisol, BHA) und di-tert-Butylmethylphenol (Butylhydroxytoluol, BHT), Ester der Gallussäure, Tocopherole (Vitamin E) sowie Ascorbinsäure und deren fettlösliche Ester. Es besteht aber ein großer Bedarf an weiteren Antioxidantien, und zwar insbesondere an solchen, die Lebensmitteln zugesetzt werden können, ohne dabei den Rechtscharakter eines gegebenenfalls zulassungspflichtigen Zusatzstoffes aufzuweisen.

Die Kajimoto, G. et al.: "Antioxidant effects of barley aqueous extract on the oxidative deterioration of oil." NIPPON EIYO SHOKURYO GAKKAISHI - JOURNAL OF JAPANESE SO-CIETY OF NUTRITION AND FOOD SCIENCE, Bd. 44, Nr. 11, 1997, Seiten 788-794, XP000866197 TOKYO., JP ISSN: 0287-3516 offenbart ein Verfahren zur Herstellung eines antioxidativ wirksamen Extraktes, bei dem geröstete (also nicht-enzymatisch gebräunte) Gerstenkörner mit Ethanol als Lösungsmittel extrahiert werden. Bei der Extraktion wird von Getreidekörnern ausgegangen.

Die GB-A-2 263 856 offenbart einen antioxidativ wirksamen Extrakt, der aus Gerstenmaische, also aus zerkleinerten Getreidekörnern, unter Verwendung von Wasser als Extraktionsmittel hergestellt und als Antioxidans in Lebensmittein eingesetzt werden kann. Auch hier bildet das gesamte Getreidekorn die Grundlage der Extraktion.

IN PATENT ABSTRACTS OF JAPAN vol. 014, no. 126 (C-0699) & JP 02 003495 A (OKUNO SEIYAKU KOGYO) wird ein doppeltes Extraktionsverfahren, bei dem zunächst geröstete Pflanzenfasern mit Wasser extrahiert werden, der wässrige Extrakt dann sprühgetrocknet und das resultierende sprühgetrocknete Produkt mit einer erneuten Extraktion mit zum Beispiel Ethanol unterzogen wird. Das nach der doppelten Extraktion erhaltene Produkt besitzt eine antioxidative Wirkung. Auch hier wird nicht von von anderen Bestandteilen des Getreidekomes abgetrennten Weizenkeimen für die Extraktion ausgegangen.

Es war die Aufgabe der vorliegenden Erfindung, ein Antioxidans anzugeben, welches insbesondere geeignet ist, lipidreiche Lebensmittel zu stabilisieren.

Diese Aufgabe wird erfindungsgemäß gelöst durch die Angabe eines antioxidativ wirksamen Extraktes, wobei der Extrakt herstellbar ist, indem man nichtenzymatisch gebräunte Getreidekeime oder eine Mischung, die nichtenzymatisch gebräunte Getreidekeime umfaßt, mit einem einen E_{T}^{N}-Wert zwischen 0,6 und 0,8 besitzenden Lösungsmittel oder Lösungsmittelgemisch extrahiert und das Extraktionsmittel gegebenenfalls abtrennt.

Zum E_{T}^{N}-Wert und seiner Bestimmung vergleiche Christian Reichhardt, Chem. Rev. 1994, 94, 2319 - 2358.

Die Getreidekeime werden vorzugsweise vor dem Röstvorgang auf übliche Weise von den sonstigen Bestandteilen des Getreidekorns, insbesondere von der Fruchtschale, der Epidermis (Samenschale, Kleie), dem Endosperm (Stärke und Kleber) und der Aleuron-Schicht getrennt, denn diese sonstigen Bestandteile nehmen im Vergleich zum Keim ein großes Volumen ein und verteuern dadurch den Röstprozeß.

Gemäß einem entsprechenden erfindungsgemäßen Verfahren zur Herstellung eines antioxidativ wirksamen Extraktes werden nichtenzymatisch gebräunte Getreidekeime oder eine Mischung, die nichtenzymatisch gebräunte Getreidekeime umfaßt, mit einem einen E_{T}^{N}-Wert zwischen 0,6 und 0,8 besitzenden Lösungsmittel oder Lösungsmittelgemisch extrahiert und das Extraktionsmittel ggf. abgetrennt.

Die mit einem solchen polaren Extraktionsmittel, z.B. mit Ethanol oder einer ethanolischen Lösung, erhaltenen Extrakte sind zur Stabilisierung lipidreicher Lebensmittel überraschenderweise geeigneter als Extrakte, die mit Extraktionsmitteln geringerer Polarität (wie z.B. Aceton oder Dieethylether) erhalten werden. A priori war dies nicht zu vermuten, denn es ist bekannt, daß polare Extraktions-Lösungsmittel in erster Linie polare Inhaltsstoffe aus dem jeweiligen Extraktionsgut extrahieren, und polare Stoffe werden aufgrund ihrer geringen Fettlöslichkeit gemeinhin als für die Stabilisierung lipidreicher Lebensmittel ungeeignet eingestuft.

Die erfindungsgemäß extrahierten Getreidekeime (Poaceae) sind vorzugsweise Weizen-, Gersten- oder sonstige Keime von Getreiden aus der unterfamilie der Pooideae; auch Maiskeime und sonstige Keime der entsprechenden anderen Getreide-Unterfamilien können mit gutem Erfolg eingesetzt werden.

Die nichtenzymatische Bräunung erfolgt typischerweise durch Rösten, und zwar vorteilhaft unter Einwirkung trockener Hitze bei einer Temperatur von vorzugsweise zwischen 50 und 250 °C; Rösttemperaturen im Bereich zwischen 120 und 170 °C sind bevorzugt und besonders bevorzugt sind Rösttemperaturen zwischen 140 und 160 °C. Eine Erhöhung der Rösttemperatur bewirkt in den genannten Temperaturbereichen allgemein eine Verbesserung der antioxidativen Wirkung des entsprechenden Extrakts (siehe dazu unten Beispiel 10). Bei einer Erhöhung der Rösttemperatur auf über 160 °C wird jedoch keine signifikante Verbesserung der antioxidativen Wirkung mehr erzielt. Bei Rösttemperaturen unter etwa 160-170 °C werden - falls überhaupt - nur vernachlässigbare Mengen an toxischen Begleitstoffen gebildet, während bei höheren Temperaturen bedenkliche Mengen an diesen Stoffen entstehen können.

Die Röstdauer beträgt vorzugsweise 5-100 Minuten.

Bei der Bräunung bilden sich Produkte der Maillard-Reaktion, und es ist nun gelungen, durch Extraktion mit den genannten Lösungsmitteln oder Lösungsmittelgemischen einen entsprechenden Extrakt zu gewinnen, der eine überraschend hohe antioxidative Wirksamkeit besitzt. Kontrollversuche haben überraschenderweise gezeigt, daß eine Fraktionierung dieses (Gesamt-)Extraktes nicht zu Substanzgemischen führt, die eine gegenüber dem unbehandelten (Gesamt-)Extrakt verbesserte antioxidative Wirksamkeit besitzen, sondern daß der gewonnene (Gesamt-)Extrakt selbst die höchste Wirksamkeit besitzt. Dies läßt sich auf einen überraschenden synergistischen Effekt der Extrakt-Inhaltsstoffe zurückführen.

Wenngleich also eine Fraktionierung nicht zu einer Verbesserung der antioxidativen Eigenschaften des erfindungsgemäßen Extraktes führt, ist es doch manchmal sinnvoll, die aroma- und/oder farbgebenden Begleitstoffe des Extraktes von diesen abzutrennen. Dem Fachmann stehen hierfür die üblichen Trennverfahren zur Verfügung.

Gemäß einem weiteren Aspekt umfaßt die Erfindung ganz allgemein die Verwendung von Extrakten nichtenzymatisch gebräunter Getreidekeime als antioxidativ wirksame Mittel zur Stabilisierung von Lebensmitteln, insbesondere lipidreichen Lebensmitteln, oder kosmetischen Mitteln, wobei zur Herstellung der Extrakte beliebige Lösungsmittel oder Lösungsmittelgemische, insbesondere solche, die bei Raumtemperatur (20-25°C) flüssig sind, als Extraktionsmittel eingesetzt werden können.

Zur Verwendung als Antioxidans besonders geeignet sind jedoch die erfindungsemäßen Extrakte, zu deren Herstellung ein Extraktionsmittel aus der insbesondere Ethanol umfassenden Gruppe der dipolar protischen Lösungsmittel eingesetzt wird, und zwar insbesondere eines mit einem E_{T}^{N}-Wert zwischen ca. 0,6 (1-Propanol) und ca. 0,8 (Glykol). Diese (polaren) Extrakte stehen überraschenderweise hinter den üblichen synthetischen Antioxidantien nicht zurück und sind ihnen sogar teilweise überlegen.

Die Erfindung betrifft auch Lebensmittel und kosmetische Mittel, die eine stabilisierend wirkende Menge eines erfindungsgemäßen Extrakts oder einer Fraktion eines solchen Extraktes umfassen.

Die erfindungsgemäßen Extrakte sind insbesondere geeignet, lipidreiche Lebensmittel wie reine handelsübliche Pflanzenöle (z.B. Maiskeimöl) oder komplexe, sensitive Lebensmittel (wie zum Beispiel ungebräunte Weizenkeime selbst) zu stabilisieren. Beispielsweise können also ungebräunte Weizenkeime stabilisiert werden, indem man einen kleinen Anteil der Weizenkeime röstet, die gerösteten Weizekeime mit Ethanol extrahiert und den Extrakt auf die ungebräunten Weizenkeime appliziert.

Überraschenderweise jedoch hat sich im Rahmen der Erfindung auch gezeigt, daß zur Stabilisierung ungebräunter Getreidekeime nicht nur der erfindungsgemäße Extrakt, sondern auch nichtenzymatisch gebräunte Getreidekeime selbst eingesetzt werden können, wobei die ungebräunten Getreidekeime mit den nichtenzymatisch gebräunten Getreidekeimen gemischt werden. Vorteilhaft ist hierbei die Einstellung eines Massen-Mischungsverhältnisses von nichtenzymatisch gebräunten zu ungebräunten Getreidekeimen im Bereich von 2 : 100 bis 8 : 100, vorzugsweise jedoch von 2 : 100 bis 4 : 100.

Nachfolgend wird die Erfindung anhand von Ausführungsbeispielen unter Bezugnahme auf die Abbildungen näher erläutert.

### Beispiel 1: Begriffsbestimmung / Rösten von Weizenkeimen

Gegenstand der nachfolgenden Untersuchungen bzw. Behandlungen waren drei unterschiedliche Probentypen von Weizenkeimen:
a) Frische Weizenkeime vom örtlichen Markt.
b) Geröstete Weizenkeime: Frische Weizenkeime wurden in einen 5-ml-Kolben mit Glasstopfen gefüllt und für 20 Minuten in einem Metallblock (Typ S-35-240, Firma Liebisch, Deutschland) bei einer Temperatur von 160° C gehalten. Anschließend wurden die so gerösteten Weizenkeime mit flüssigem Stickstoff schockgekühlt und in nachfolgenden Untersuchungen eingesetzt.
c) Geröstete entfettete Weizenkeime: Frische Weizenkeime wurden entfettet, indem man sie für 14 bis 16 Stunden einer Soxhlet-Extraktion mit n-Hexan unterzog. Danach wurden die so entfetteten Weizenkeime wie unter b) geröstet und weiterbehandelt.

### Beispiel 2: Extraktion gerösteter Weizenkeime mit verschiedenen Extraktions-Lösungsmitteln

Frische Weizenkeime wurden gemäß Beispiel 1 b) geröstet. Je 40 g der erhaltenen gerösteten Weizenkeime wurde mehrmals kalt mit insgesamt 100 bis 300 ml der Extraktions-Lösungsmittel (a) Diethylether, (b) Aceton bzw. (c) Ethanol extrahiert, so daß sich insgesamt drei Extrakte ergaben. Jeder dieser drei Extrakte wurde unter Verwendung eines Rotationsverdampfers bei 35 °C im Vakuum aufkonzentriert, so daß das verbleibende Extrakt-Volumen 20 ml betrug. In 1 ml Extrakt sind somit die Inhaltsstoffe von 2 g gerösteten Weizenkeimen enthalten. Jeder der aufkonzentrierten Extrakte wurde quantitativ in einen 20 ml-Kolben überführt und zur weiteren Verwendung im Dunkeln bei -30 °C gelagert.

### Anmerkung:

In den nachfolgenden Beispielen wird die Dosierung von Antioxidantien bei der Behandlung von Lebensmitteln in Prozent angegeben. Soweit sich die Dosierungsangaben auf Extrakte gerösteter Weizenkeime beziehen, sind sie wie folgt zu verstehen:
Eine z.B. 10 %ige Dosierung bedeutet, daß der Extrakt von 10 g Weizenkeimen zu 100 g Lebensmittel (z.B. Weizenkeime, Maisöl, Weizenöl) gegeben wurde. Dies entspricht nach der obigen Vorschrift einer Zugabe von 5 ml aufkonzentriertem Extrakt.

### Beispiel 3: Vergleichender Test zur Stabilisierung von Weizenkeimöl mit verschiedenen Antioxidantien

### 3.1. Es wurden zunächst insgesamt 4 Proben von je 50 g Weizenkeimöl einem Stabilisierungstest unterzogen.

Die Proben wurden dazu in offene Becher mit einem Durchmesser von jeweils 8,6 cm gefüllt. Unter Rühren für 10 Minuten wurden dann drei der Proben mit unterschiedlichen Antioxidantien versetzt, und zwar mit
- Ascorbylpalmitat (0,02 Gew.-%) als Beispiel für einen antioxidativ wirksamen Metallchelat-Bildner,
- BHA (0,02 Gew.-%) als Beispiel für ein phenolisches Antioxidans bzw.
- einem erfindungsgemäßen ethanolischen Extrakt gemäß Beispiel 2c (20 %, siehe Anmerkung zu Beispiel 2).
- Die vierte Probe wurde nicht mit einem Antloxidans versetzt und diente Kontrollzwecken.

Die Proben wurden bei 50 °C gelagert.

### 3.2. Der Stabilisierungstest wurde unter ansonsten gleichen Bedingungen, aber mit einer Lagertemperatur von 60 °C, wiederholt.

Die oxidative Stabilität der verschiedenen Proben (gemäß 3.1 und 3.2) wurde durch wiederholte Analyse ermittelt, wobei im 24-Stunden-Takt jeweils der Peroxid-Wert, die Konzentration an konjugierten Dien-Hydroperoxiden und die Konzentration an α-Tocopherol bestimmt wurde. Der Peroxid-Wert und die Hydroperoxid-Konzentration sind dabei empirische Maße für die Lipid-Oxidation.

Die Ergebnisse der Bestimmungen sind in den Fig. 1-6 wiedergegeben:

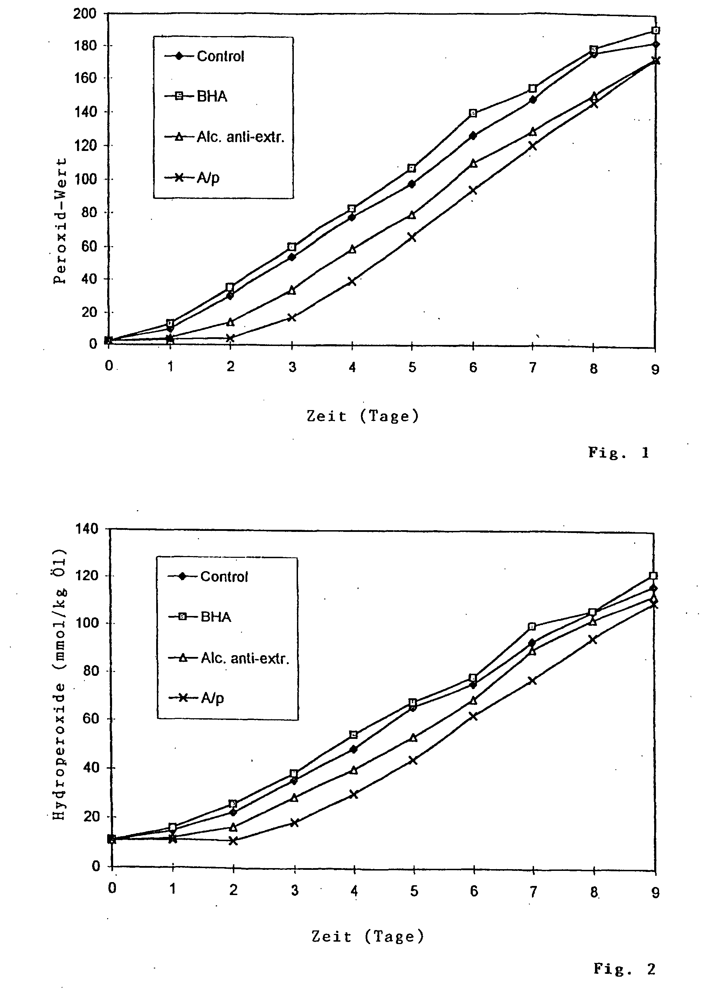

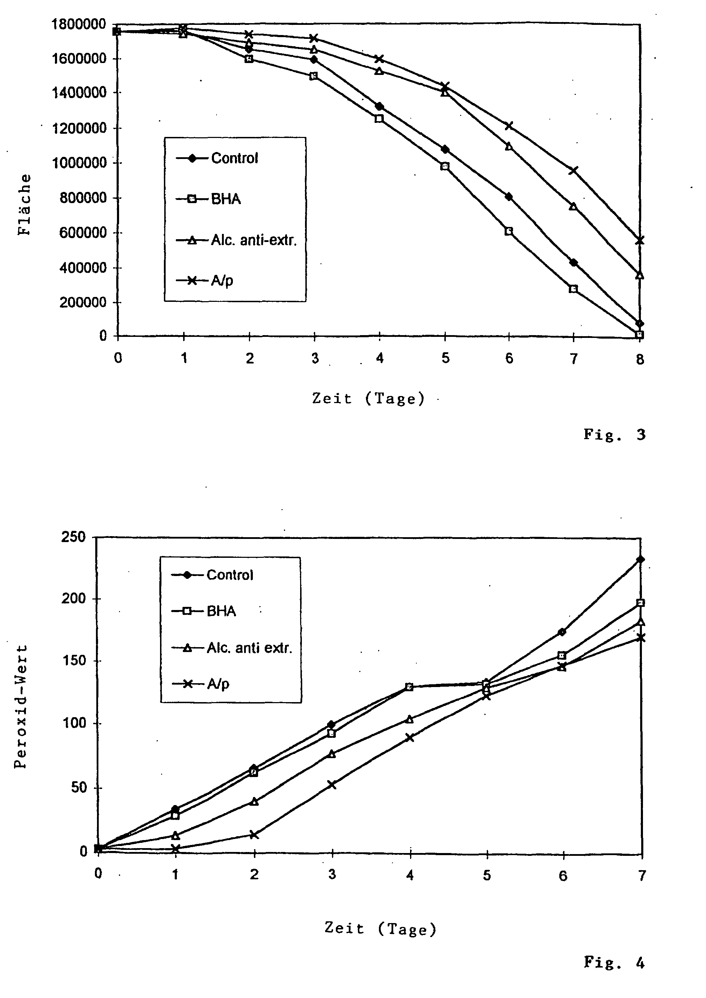

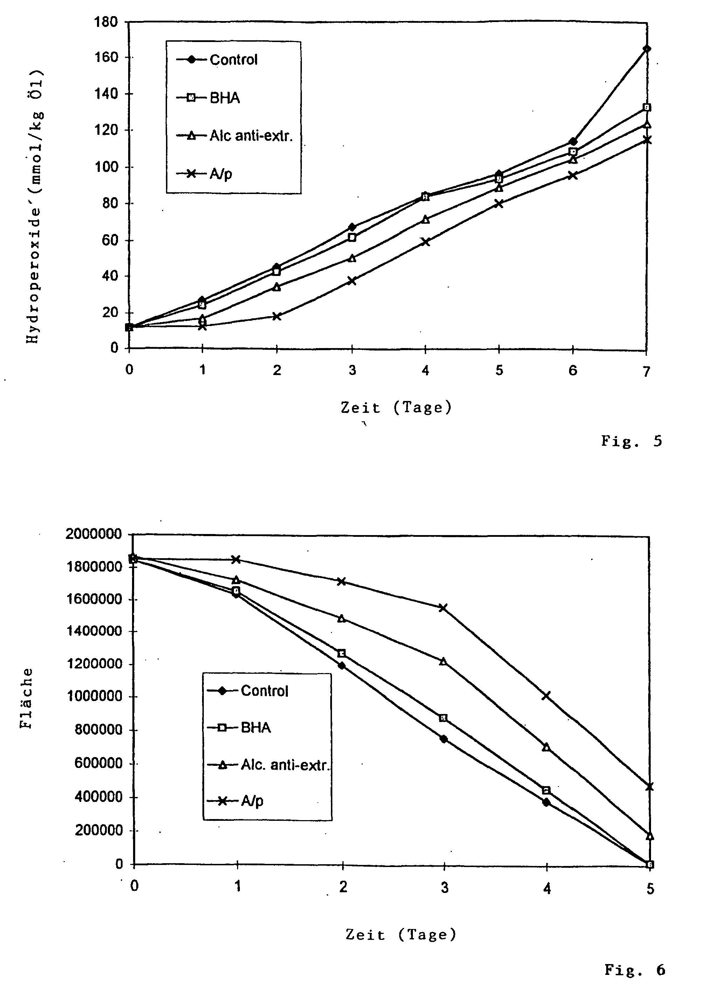

Es stellen dar:
- Fig. 1: Peroxid-Werte handelsüblichen Weizenkeimöls, das bei 50 °C mit unterschiedlichen Antioxidantien gelagert wurde.
- Fig. 2: Konzentration an konjugierten Dien-Hydroperoxiden In handelsüblichem Weizenkeimöl, das bei 50 °C mit unterschiedlichen Antioxidantien gelagert wurde.
- Fig. 3: α-Tocopherol-Konzentration in handelsüblichem Weizenkeimöl, das bei 50 °C mit unterschiedlichen Antioxidantien gelagert wurde.
- Fig. 4: Peroxid-Werte handelsüblichen Weizenkeimöls, das bei 60 °C mit unterschiedlichen Antioxidantien gelagert wurde.
- Fig. 5: Konzentration an konjugierten Dien-Hydroperoxiden in handelsüblichem Weizenkeimöl, das bei 60 °C mit unterschiedlichen Antioxidantien gelagert wurde.
- Fig. 6: α-Tocopherol-Konzentration in handelsüblichem Weizenkeimöl, das bei 60 °C mit unterschiedlichen Antioxidantien gelagert wurde.
(Control = Kontrollprobe; BHA = Butyl-Hydroxanisol; Alc. anti-extr. = erfindungsgemäßer ethanolischer Extrakt gemäß Beispiel 2c; A/p = Ascorbylpalmitat)
- Wie sich aus dem Vergleich der Fig. 1-3 mit den Fig. 4-6 ergibt, war die Oxidationsgeschwindigkeit bei 60 °C erwartungsgemäß höher als bei 50 °C, und die Induktionszeit war bei 60 °C kürzer.
- Die Bestimmung des Peroxid-Werts zeigte, daß der 20 %ige ethanolische Extrakt gerösteter Weizenkeime einen antioxidativen Effekt besaß, der besser als der von BHA, aber geringfügig schlechter als der von Ascorbylpalmitat war (vgl. die Fig. 1 und 4, in denen die Zunahme des Peroxid-Werts einer Zunahme von Autoxidations-Produkten im Weizenkeimöl entspricht).
- Die Messungen zur Konzentration konjugierter Dien-Hydroperoxide im Weizenkeimöl entsprachen den Resultaten aus der Bestimmung des Peroxid-Wertes (vgl. die Fig. 2 und 5, in denen die Zunahme der Hydroperoxid-Konzentration einer Zunahme von Autoxidations-Produkten im Weizenkeimöl entspricht).
- Die in den Fig. 3 und 6 abgebildete Veränderung der α-Tocopherol-Konzentration in Weizenkeimöl, die als Indikator für dessen Stabilität dient, bestätigte ebenfalls die Ergebnisse der Untersuchungen zum Peroxid-Wert und zur Konzentration konjugierter Dien-Hydroperoxide. Eine Abnahme der α-Tocopherol-Konzentratlon ist dabei analog einer Zunahme der Peroxid-Werte und der Konzentration an konjugierten Dien-Hydroperoxiden zu bewerten.
- Rancimat-Messungen bestätigten schließlich nochmals die Ergebnisse aus den in den Figuren zusammengefaßten Meßreihen.

### Beispiel 4: Vergleichender Test zur Stabilisierung tocopherolfreien Maisöls mit verschiedenen Antioxidantien

Es wurden vier Proben zu je 50 g tocopherolfreies Maisöl ("stripped corn oil") untersucht. Diese Proben wurden in offene Becher mit einem Durchmesser von jeweils 8,6 cm gefüllt. Unter Rühren für 10 Minuten wurden dann drei der Proben mit unterschiedlichen Antioxidantien versetzt, und zwar mit Ascorbylpalmitat (0,02 Gew.-%), BHA (0,02 Gew.-%) bzw. einem ethanolischen Extrakt gemäß Beispiel 2c (20 %). Die vierte Probe wurde nicht mit einem Antioxidans versetzt und diente Kontrollzwecken.

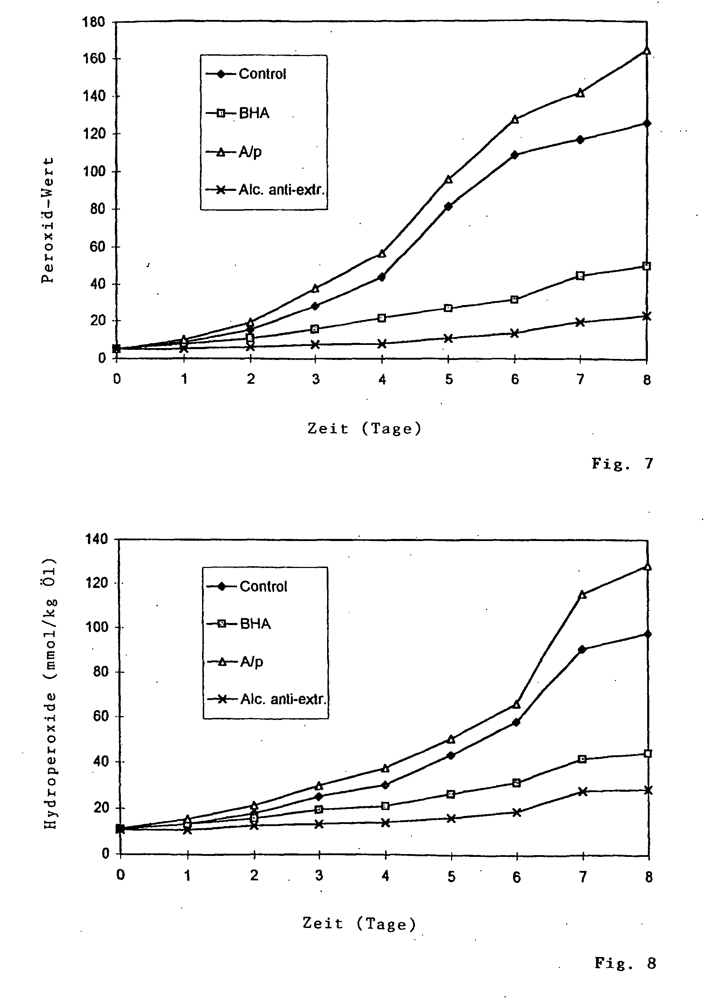

Die Proben wurden bei 60 °C gelagert. Die oxidative Stabilität der verschiedenen Proben wurde durch wiederholte Analyse ermittelt, wobei im 24-Stunden-Takt jeweils der Peroxidwert und die Konzentration an konjugierten Dien-Hydroperoxiden bestimmt wurde, vgl. die Fig. 7 und 8.

Es stellen dar:
- Fig. 7: Peroxidwerte in tocopherolfreiem Maisöl, das bei 60 °C mit unterschiedlichen Antioxidantien gelagert wurde.
- Fig. 8: Konzentration an konjugierten Dien-Hydroperoxiden in tocopherolfreiem Maisöl, das bei 60 °C mit unterschiedlichen Antioxidantien gelagert wurde.
(Control = Kontrollprobe; BHA = Butyl-Hydroxanisol; Alc. anti-extr. = erfindungsgemäßer ethanolischer Extrakt gemäß Beispiel 2c; A/p = Ascorbylpalmitat)

Beide Meßmethoden führten zu dem Ergebnis, daß die antioxidative Wirkung des 20 %igen ethanolischen Extraktes gerösteter Weizenkeime größer war als der von BHA und Ascorbylpalmitat. Geht man von der in Fig. 8 dargestellten Untersuchung der Hydroperoxid-Bildung aus, so zeigt Ascorbylpalmitat (A/p) beim gegebenen Konzentrationsniveau sogar einen pro-oxidativen Effekt.

### Beispiel 5: Vergleichender Test zur Stabilisierung von Weizenkeimen mit verschiedenen Antioxidantien

Weizenkeime vom örtlichen Markt sind in der Regel vor-stabilisiert (behandelt), indem sie mit heißer Luft oder heißem Wasserdampf erwärmt werden, um die in ihnen enthaltenen Enzyme zu inaktivieren, welche ansonsten für das Verderben von Weizenkeimen mitverantwortlich sind. Vier Ansätze zu je 500 g der so vor-stabilisierten (behandelten) frischen Weizenkeime sowie-parallel dazu - vier entsprechende Ansätze frischer, unbehandelter Weizenkeime gemäß Beispiel 1 a) wurden mit (a) 20 ml Ethanol (zu Kontrollzwekken), (b) 20 ml einer 0,01 % BHA-Lösung in Ethanol, (c) 20 ml einer 0,01 % Ascorbylpalmitat-Lösung in Ethanol bzw. (d) 20 ml einer 8 %igen Lösung des ethanolischen Extraktes gerösteter Weizenkeime gemäß Beispiel 2 besprüht. Jeder der insgesamt acht Ansätze wurde in 2 Schalen ausgebreitet, und zwar so, daß die Höhe der Weizenkeim-Schichten in keiner Schale höher als 1 cm war. Die Ansätze wurden bei 50 °C gelagert.

Unter Verwendung einer üblichen Soxhlet-Apparatur und mit Pentan/Dichlormethan (2:1) als Extraktionsmittel wurden aus den Ansätzen im wöchentlichen Abstand Weizenkeimöl-Proben extrahiert. Die Keimöl-Extrakte wurden gefiltert, 24 Stunden über wasserfreiem Natriumsulfat getrocknet und dann wieder gefiltert. Das Lösungsmittel wurde unter Hochvakuum bei 35 °C entfernt. Das resultierende rohe Öl wurde unmittelbar untersucht.

Die oxidative Stabilität der Weizenkeime wurde analysiert, indem für die erhaltenen Öl-Proben der Peroxid-Wert, die Konzentration konjugierter Dien-Hydroperoxide und die Konzentration an α-Tocopherol bestimmt wurden.

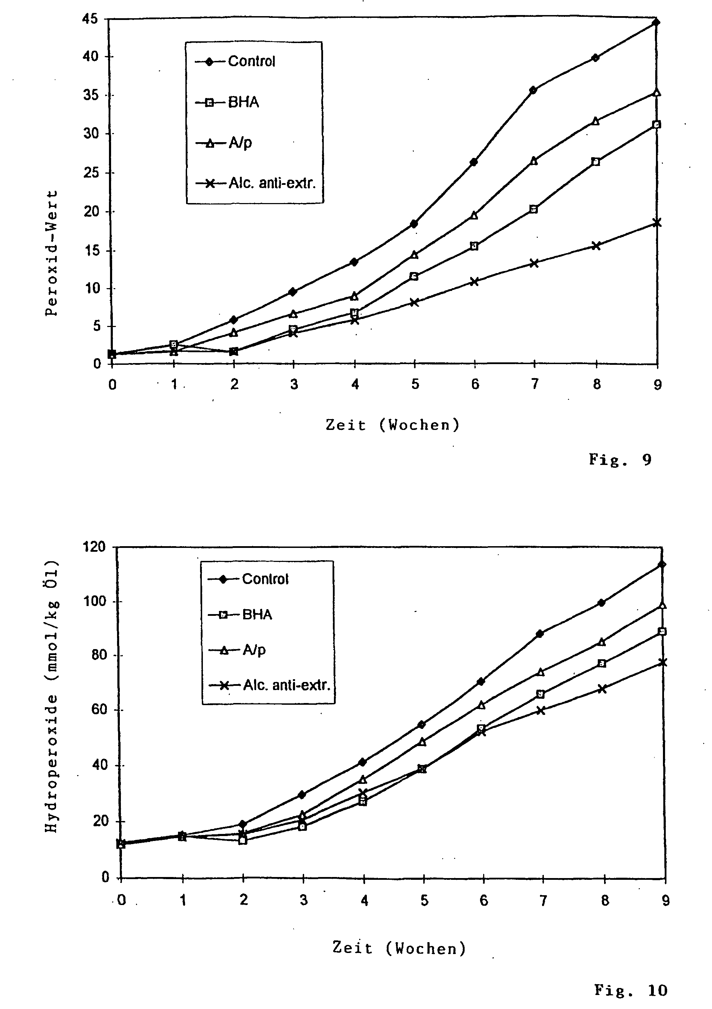

Die in den Figuren 9 bis 14 dargestellten Ergebnisse zeigen, daß der ethanolische Extrakt gerösteter Weizenkeime zur Stabilisierung sowohl von behandelten als auch von unbehandelten Weizenkeimen besser geeignet war als die üblichen Antioxidantien BHA und Ascorbylpalmitat.

Es stellen dar:
- Fig. 9: Peroxid-Werte behandelter Weizenkeime, die bei 50 °C mit unterschiedlichen Antioxidantien gelagert wurden.
- Fig. 10: Konzentration an konjugierten Dien-Hydroperoxiden in behandelten Weizenkeimen, die bei 50 °C mit unterschiedlichen Antioxidantien gelagert wurden.

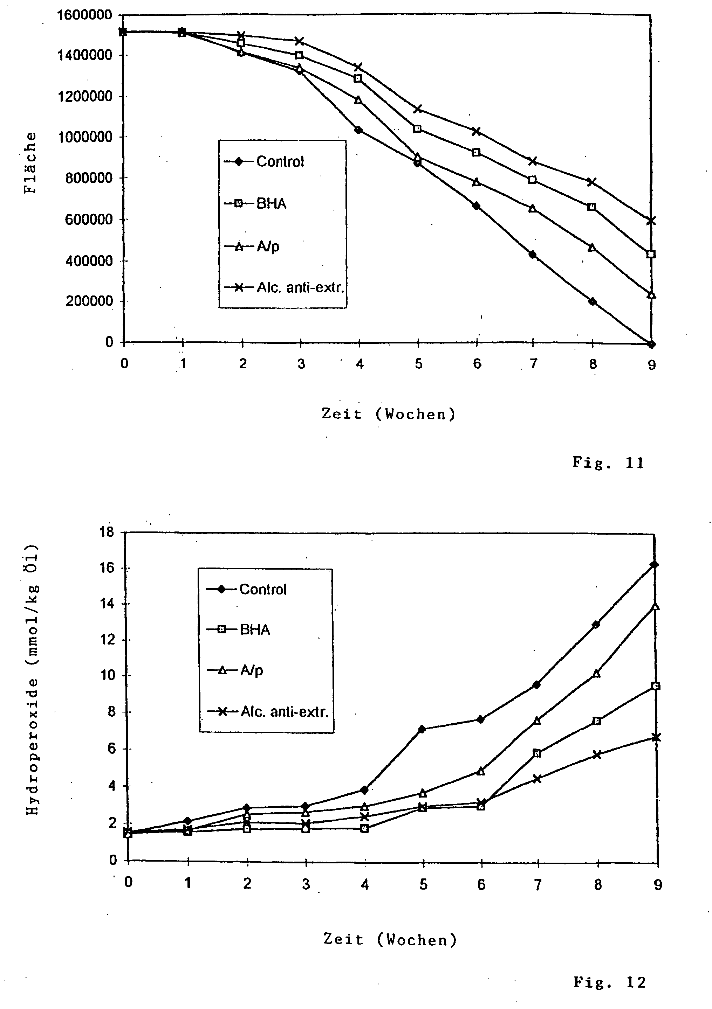

- Fig. 11: α-Tocopherol-Konzentration in behandelten Weizenkeimen, die bei 50 °C mit unterschiedlichen Antioxidantien gelagert wurden.
- Fig. 12: Peroxid-Werte unbehandelter Weizenkeime, die bei 50 °C mit unterschiedlichen Antioxidantien gelagert wurden.

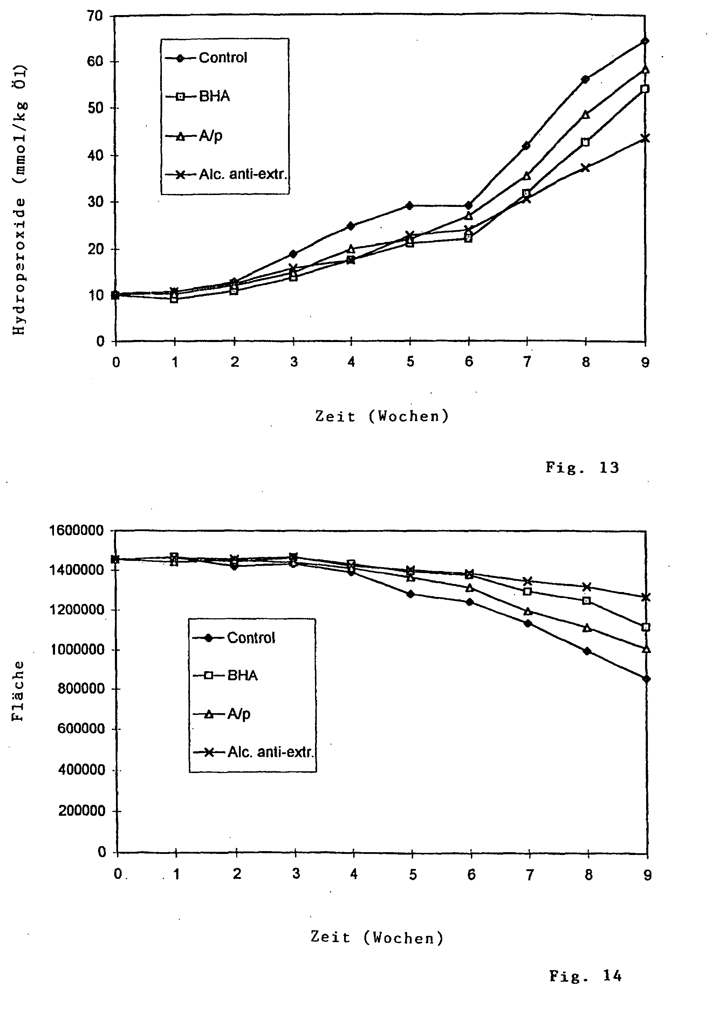

- Fig. 13: Konzentration an konjugierten Dien-Hydroperoxiden in unbehandelten Weizenkeimen, die bei 50 °C mit unterschiedlichen Antioxidantien gelagert wurden.
- Fig. 14: α-Tocopherol-Konzentration in unbehandelten Weizenkeimen, die bei 50 °C mit unterschiedlichen Antioxidantien gelagert wurden.
(Control = Kontrollprobe; BHA = Butyl-Hydroxanisol; Alc. anti-extr. = erfindungsgemäßer ethanolischer Extrakt gemäß Beispiel 2c; A/p = Ascorbylpalmitat)

### Beispiel 6: Vergleichender Test zur Stabilisierung von Maisöl mit verschiedenen Extrakten gerösteter Weizenkeime

Es sollte nachgewiesen werden, daß mit Extraktions-Lösungsmitteln unterschiedlicher Polarität eine unterschiedliche antioxidative Wirkung des entsprechenden Extrakts gerösteter Weizenkeime erreicht wird. Dazu wurde die stabilisierende Wirkung von Extrakten dreier lebensmittelrechtlich zulässiger Extraktions-Lösungsmittel unterschiedlicher Polarität auf Maisöl (als Beispiel eines Pflanzenöls) getestet, und zwar bei Konzentrationsniveaus von 10 % und 20 %:

Insgesamt 6 Maisöl-Proben wurden mit 10 bzw. 20 % eines Diethylether-, Aceton- bzw. Ethanol-Extrakts gerösteter Weizenkeime behandelt; die Herstellung dieser Extrakte ist in Beispiel 2 beschrieben. Die oxidative Stabilität der bei 60 °C gelagerten Proben wurde durch wiederholte Analyse ermittelt, wobei im 24-Stunden-Takt jeweils der Peroxidwert, die Konzentration an konjugierten Dien-Hydroperoxiden und die Konzentration an α-Tocopherol bestimmt wurde.

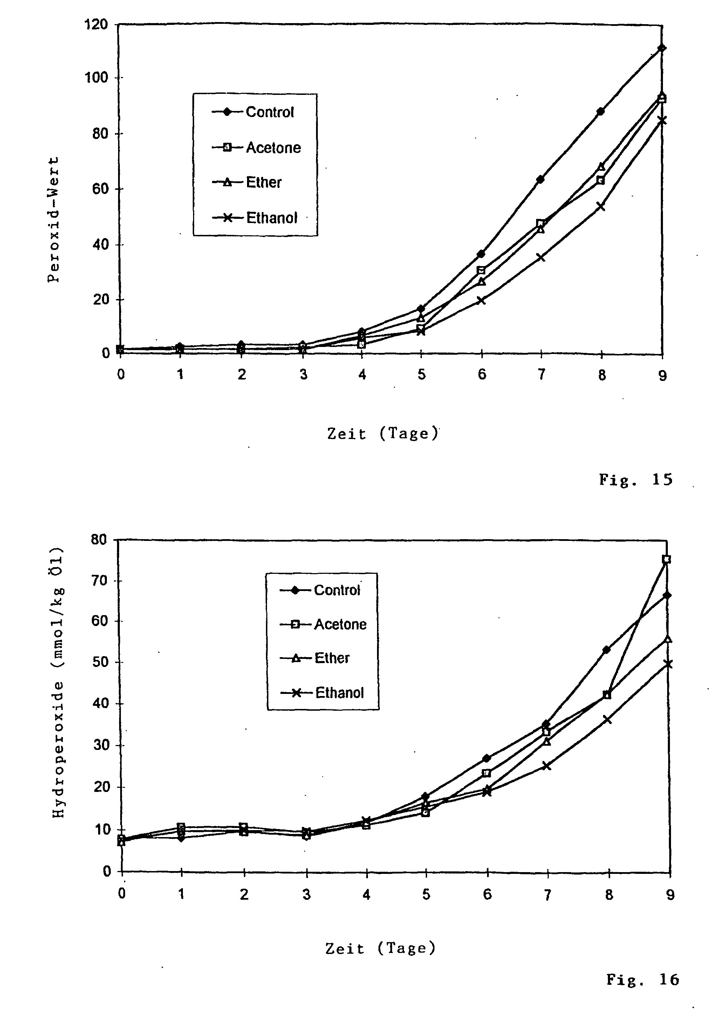

Beim Konzentrationsniveau von 10 % waren die antioxidativen Effekte der Ether- und Aceton-Extrakte recht ähnlich, aber jeweils geringer als der antioxidative Effekt des ethanolischen Extraktes, wie sich aus den nachfolgenden Fig. 15 - 17 ergibt, in denen die Resultate zum Peroxid-Wert, zur Konzentration an konjugierten Dien-Hydroperoxiden und zur Konzentration an Tocopherol angegeben sind.

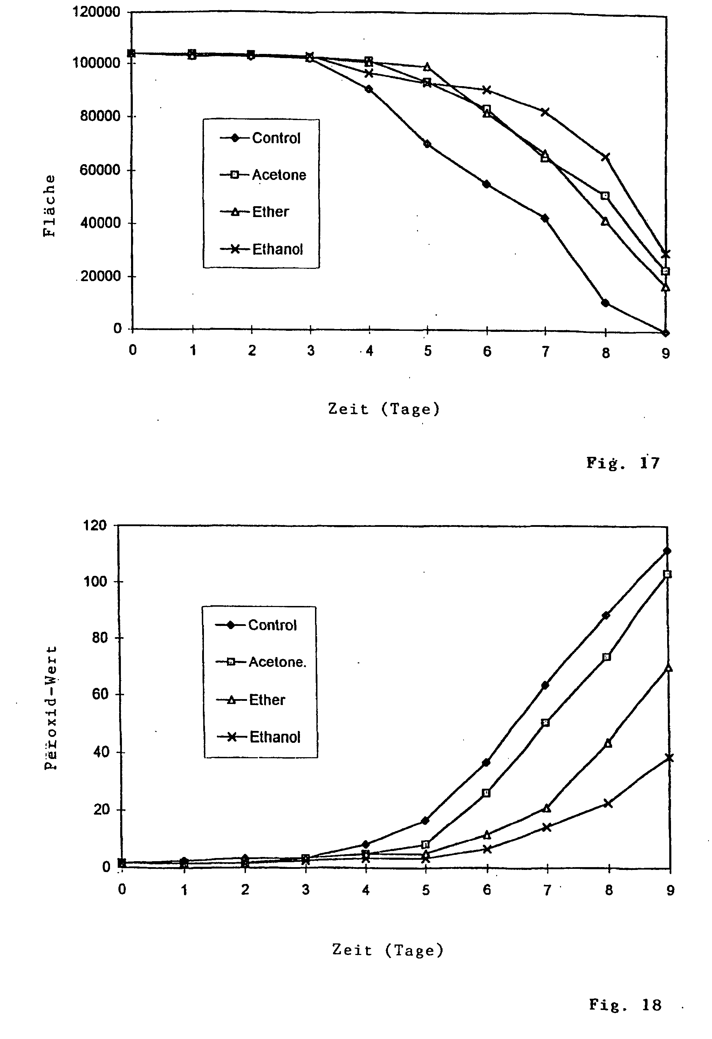

Der überlegene antioxidative Effekt des ethanolischen Extrakts gerösteter Weizenkeime war beim Konzentrationsniveau von 20 % noch ausgeprägter. Dies wird aus den Fig. 18 - 20 ersichtlich.

Es stellen dar:
- Fig. 15: Peroxid-Wert von Maisöl, das mit jeweils 10 % unterschiedlicher Lösungmittelextrakte versetzt und bei 60 °C gelagert wurde.
- Fig. 16: Konzentration an konjugierten Dien-Hydroperoxiden in Maisöl, das mit jeweils 10 % unterschiedlicher Lösungmittelextrakte versetzt und bei 60 °C gelagert wurde.
- Fig. 17: α-Tocopherol-Konzentration in Maisöl, das mit jeweils 10 % unterschiedlicher Lösungmittelextrakte versetzt und bei 60 °C gelagert wurde.
- Fig. 18: Peroxid-Wert von Maisöl, das mit jeweils 20 % unterschiedlicher Lösungmittelextrakte versetzt und bei 60 °C gelagert wurde.

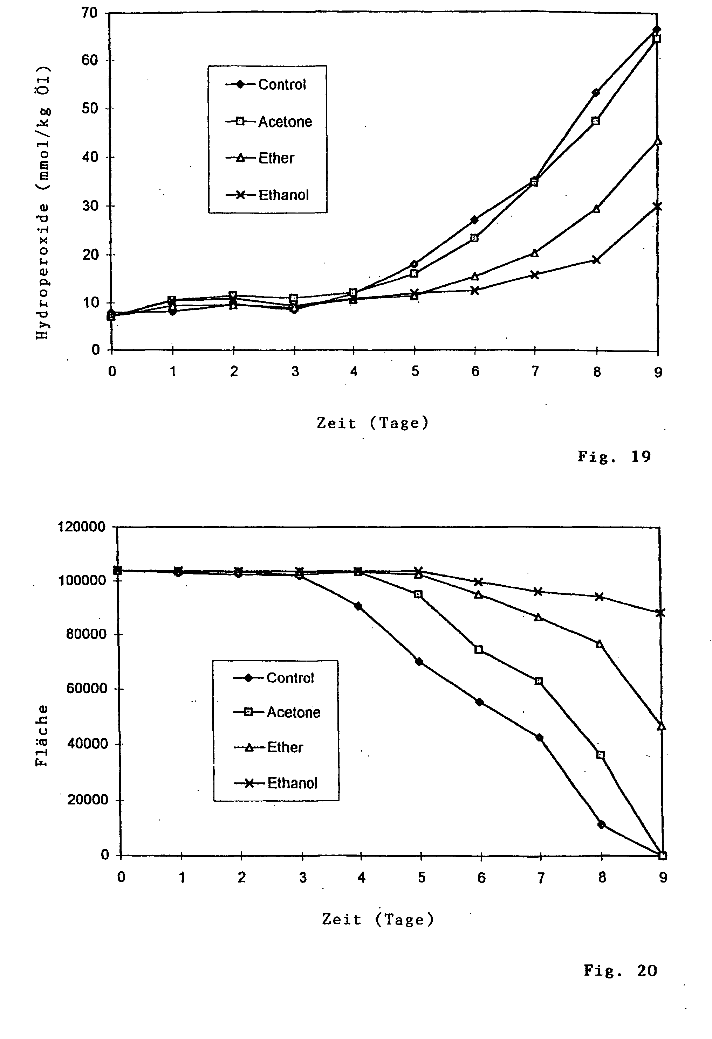

- Fig. 19: Konzentration an konjugierten Dien-Hydroperoxiden in Maisöl, das mit jeweils 20 % unterschiedlicher Lösungmittelextrakte versetzt und bei 60 °C gelagert wurde.
- Fig. 20: α-Tocopherol-Konzentration in Maisöl, das mit jeweils 20 % unterschiedlicher Lösungmittelextrakte versetzt und bei 60 °C gelagert wurde.
(Control = Kontrollprobe)

### Beispiel 7: Test zur Stabilisierung unbehandelter Weizenkeime durch Vermischen mit gerösteten Weizenkeimen

Vier Proben zu 500 g frischer unbehandelter Weizenkeime wurden mit 0 Gew.-%, 4 Gew.-%, 8 Gew.-% bzw. 16 Gew.-% gerösteter Weizenkeime vermischt.

Eine weitere 500 g-Probe von frischen Weizenkeimen wurde in zehn 50 g-Proben aufgeteilt, die dann jeweils in eine Petrischale von 19 cm Durchmesser überführt und für 5 Minuten einer Mikrowellenbehandlung bei 600 Watt ausgesetzt wurden. Die zehn 50 g-Proben wurden dann wieder vereinigt.

Jede der somit insgesamt 5 Proben zu 500 g wurde in 2 Schalen ausgebreitet und bei 50 °C gelagert.

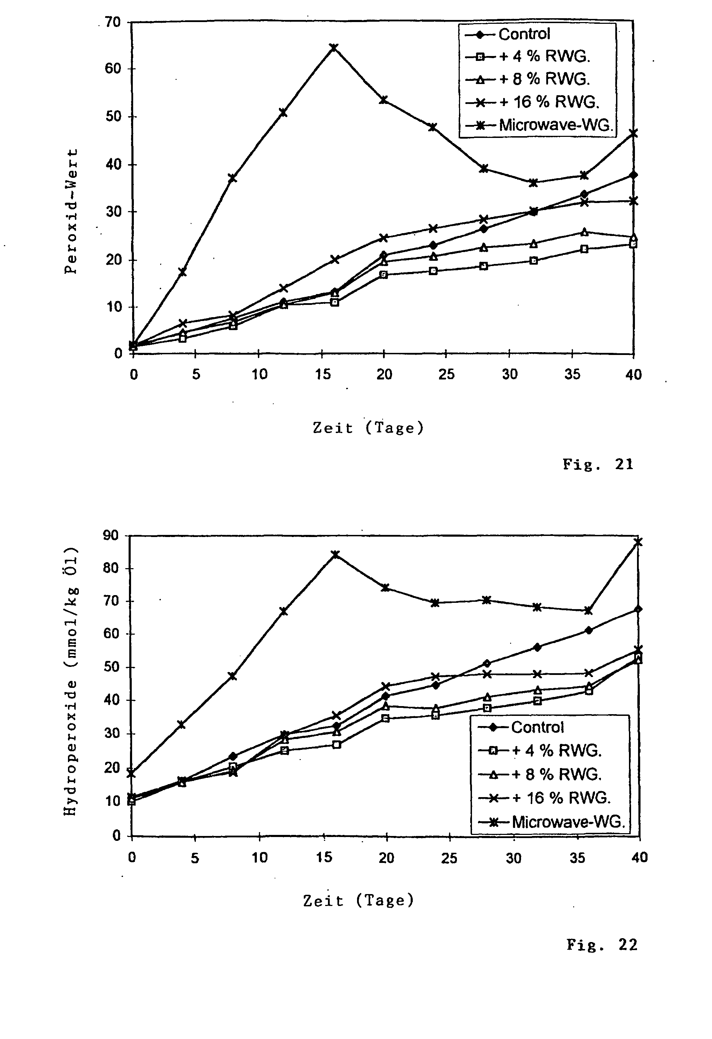

Die oxidative Stabilität der Proben wurde durch wiederholte Analyse ermittelt, wobei im 24-Stunden-Takt jeweils der Peroxid-Wert, die Konzentration an konjugierten Dien-Hydroperoxiden und die Konzentration an α-Tocopherol bestimmt wurde, vgl. die Fig. 21 - 23.

Die Stabilisierung von Weizenkeimen durch Zugabe kleiner Mengen gerösteter Weizenkeime war wirksamer als eine Mikrowellenbehandlung, welche bekanntlich die lipolytischen Enzyme eines Keims stabilisieren soll. Die Zugabe von 4 % gerösteten Weizenkeimen hatte bei 50 °C eine bessere antioxidative Wirkung als die Zugabe von 8 % oder 16 % gerösteten Weizenkeimen. Bei Zugabe von 16 % gerösteten Weizenkeimen wurde sogar innerhalb der ersten 30 Tage ein pro-oxidativer Effekt festgestellt.

Der Test wurde mit einer weiteren Gruppe von Proben wiederholt, wobei jeweils 500 g frischer unbehandelter Weizenkeime mit 0 Gew.-%, 2 Gew.-%, 4 Gew.-%, 6 Gew.-% bzw. 8 Gew.-% gerösteter Weizenkeime vermischt wurden. Unter ansonsten gleichen Versuchs- und Auswertungsbedingungen wurden die Proben jedoch bei 40 °C gelagert.

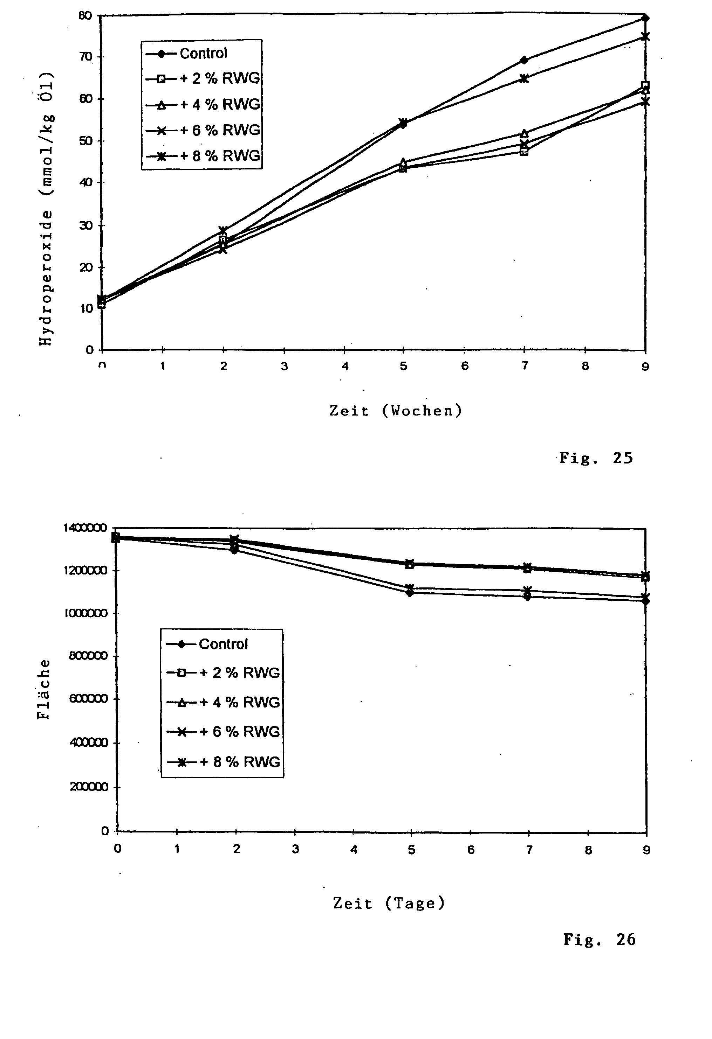

Wie sich aus den Fig. 24 - 26 ergibt, hatte bei einer Lagertemperatur von 40 °C die Zugabe von 2 %, 4 % und 6 % gerösteten Weizenkeimen einen annähernd gleichen stabilisierenden Effekt auf die frischen Weizenkeime. Bei Zugabe von 8 % gerösteter Weizenkeime war der antioxidative Effekt jedoch nicht mehr so ausgeprägt wie bei den niedrigeren Konzentrationen.

Es stellen dar:
- Fig. 21: Peroxid-Werte von Weizenkeimen, die bei 50 °C mit unterschiedlichen Konzentrationen gerösteter Weizenkeime gelagert wurden.
- Fig. 22: Konzentration an konjugierten Dien-Hydroperoxiden in Weizenkeimen, die bei 50 °C mit unterschiedlichen Konzentrationen gerösteter Weizenkeime gelagert wurden.

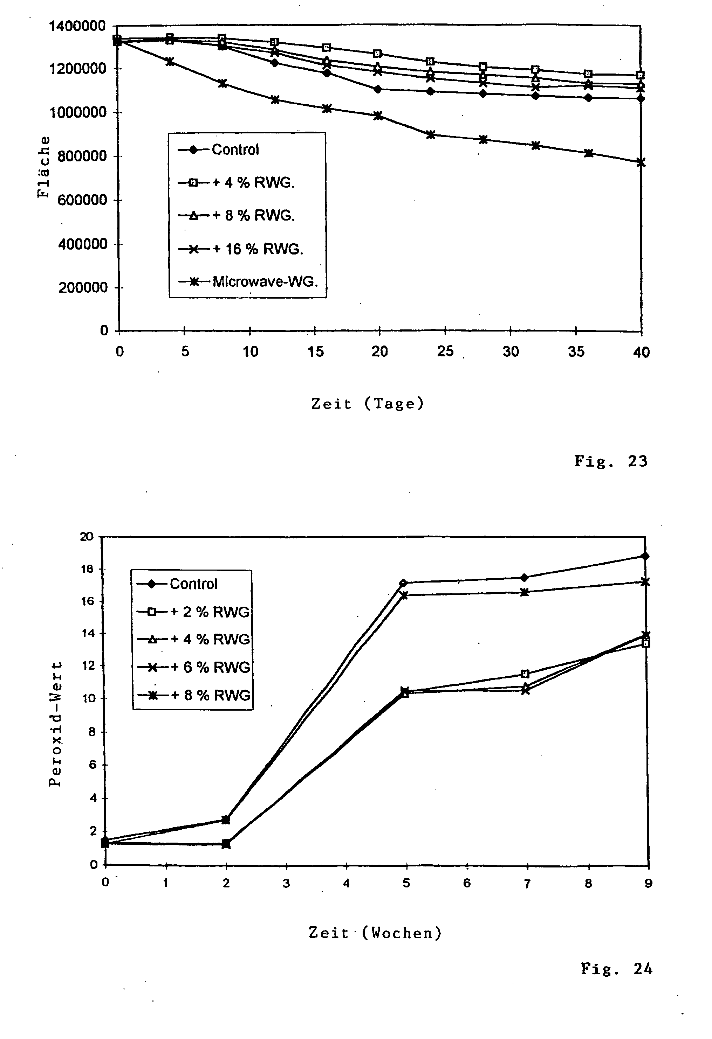

- Fig. 23: α-Tocopherol-Konzentration in Weizenkeimen, die bei 50 'C mit unterschiedlichen Konzentrationen gerösteter Weizenkeime gelagert wurden.
- Fig. 24: Peroxid-Werte von Weizenkeimen, die bei 40 °C mit unterschiedlichen Konzentrationen gerösteter Weizenkeime gelagert wurden.
- Fig. 25: Konzentration an konjugierten Dien-Hydroperoxiden in Weizenkeimen, die bei 40 °C mit unterschiedlichen Konzentrationen gerösteter Weizenkeime gelagert wurden.
- Fig. 26: α-Tocopherol-Konzentration in Weizenkeimen, die bei 40 °C mit unterschiedlichen Konzentrationen gerösteter Weizenkeime gelagert wurden.
(RWG = geröstete Weizenkeime; Microwave-WG = mikrowellenbehandelte Weizenkeime)

### Beispiel 8: Präparative HPLC-Fraktionierung des ethanolischen Extrakts gerösteter, frischer bzw. entfetteter Weizenkeime

Es wurden zwei ethanolische Extrakte gerösteter Weizenkeime hergestellt.

Der erste ethanolische Extrakt stammte dabei von gerösteten frischen Weizenkeimen gemäß Beispiel 1 b; der zweite ethanolische Extrakt stammte von gerösteten, entfetteten Weizenkeimen gemäß Beispiel 1 c.

Diese zwei Extrakte wurden mittels präparativer HPLC unter Verwendung einer Diol-Kolonne (Lichrosorb, 250 × 25 ml, Partikelgröße 7 µm, Merck, Darmstadt, Deutschland) fraktioniert. Das Elutionssystem war jeweils Dichlormethan (I) und Methanol (II). Der Gradient betrug dabei jeweils 100 % I für 10 Minuten und wurde dann durch lineare Erhöhung des Anteils an II bis zur 100. Minute auf 50 % II gebracht; nach weiteren 10 Minuten wurde er jeweils auf 100 % II erhöht. Bei diesem Gradienten wurde das System bis zur 130. Minute gehalten. Die Durchflußrate betrug 10 ml min⁻¹, und das Injektionsvolumen betrug 2 l.

Das jeweilige Eluat wurde in einem Fraktionensammler aufgefangen. Es wurden jeweils insgesamt 6 Fraktionen erhalten, und zwar je eine Fraktion für den Zeitraum von 0 - 32 Minuten (1), 32 - 44 Minuten (2), 44 - 56 Minuten (3), 56 - 76 Min (4), 76 - 86 Minuten (5) und 86 - 130 Minuten (6). Die Fraktionen wurden im Vakuum bei 40 °C aufkonzentriert und quantitativ in 5 ml-Meßkolben überführt. Es wurde jeweils soweit möglich mit Ethanol aufgefüllt. Die Fraktionen wurden bis zur Verwendung im Vergleichstest gemäß Beispiel 9 bei -30 °C gelagert.

### Beispiel 9: Vergleichstest zum antioxidativen Effekt der Fraktionen aus der Fraktionierung gemäß Beispiel 8

Die ethanolischen Fraktionen gemäß Beispiel 8 wurden auf ihre stabilisierende Wirkung untersucht. Als zu stabilisierendes Lebensmittel wurde Maisöl eingesetzt, und zwar einerseits tocopherolfreies Maisöl und andererseits Maisöl mit einem natürlichen Anteil an Tocopherol.
1. Es wurden zunächst Proben zu jeweils 10 g tocopherolfreien Maisöls ("stripped corn oil") eingesetzt. Zu diesen Proben wurden jeweils 2,5 ml aus einer der 6 Fraktionen des ethanolischen Extrakts gerösteter frischer Weizenkeime bzw. gerösteter entfetteter Weizenkeime gegeben. Zu Kontrollzwecken wurde eine 1 ml-Probe des ursprünglichen ethanolischen Extrakts gerösteter Weizenkeime (entweder frisch, d.h. fetthaltig oder entfettet) mit 1,5 ml Ethanol versetzt, und eine weitere Kontroll-Probe enthielt 2,5 ml Ethanol. Die Proben wurden jeweils für 10 Minuten gerührt.
   Anschließend wurden die Proben bei 60 °C gelagert.
   Die oxidative Stabilität wurde durch wiederholte Analyse ermittelt, wobei im 24-Stunden-Takt jeweils die Konzentration an konjugierten Dien-Hydroperoxiden bestimmt wurde.
2. Der Vergleichstest wurde mit tocopherolhaltigem Maisöl wiederholt, um auch insoweit die Stabilität in Gegenwart der Fraktionen zu testen.

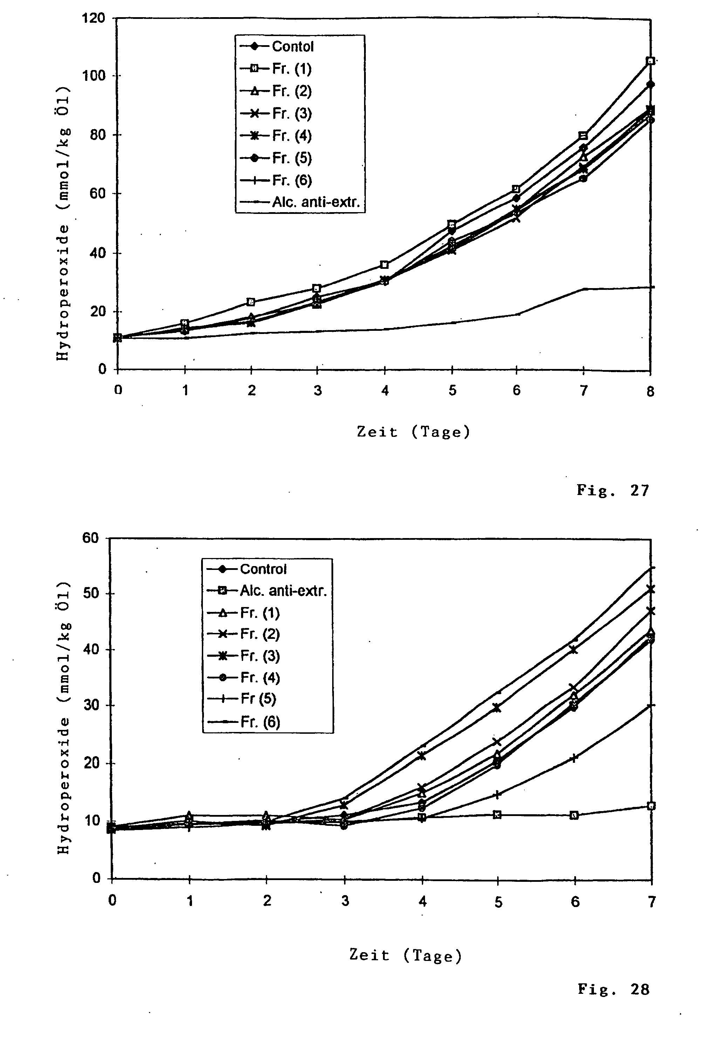

Die Ergebnisse der Untersuchungen sind in den Figuren 27 - 30 zusammengefaßt.

Es stellen dar:
- Fig. 27: Konzentration an konjugierten Dien-Hydroperoxiden in *tocopherolfreiem* Maisöl, das mit den Fraktionen 1-6 aus dem ethanolischen Extrakt gerösteter, *frischer* Weizenkeime versetzt und bei 60 °C gelagert wurde.
- Fig. 28: Konzentration an konjugierten Dien-Hydroperoxiden in *tocopherolhaltigem* Maisöl, das mit den Fraktionen 1-6 aus dem ethanolischen Extrakt gerösteter, *frischer* Weizenkeime versetzt und bei 60 °C gelagert wurde.

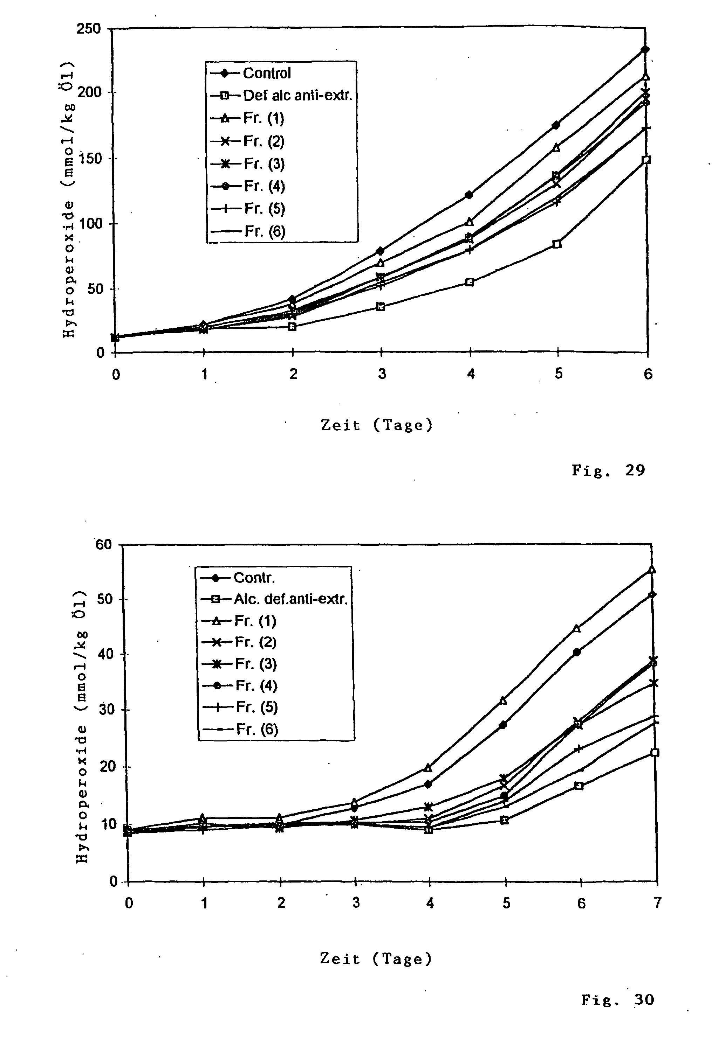

- Fig. 29: Konzentration an konjugierten Dien-Hydroperoxiden in *tocopherolfreiem* Maisöl, das mit den Fraktionen 1-6 aus dem ethanolischen Extrakt gerösteter, *entfetteter* Weizenkeime versetzt und bei 60 °C gelagert wurde.
- Fig. 30: Konzentration an konjugierten Dien-Hydroperoxiden in *tocopherolhaltigem* Maisöl, das mit den Fraktionen 1-6 aus dem ethanolischen Extrakt gerösteter, *entfetteter* Weizenkeime versetzt und bei 60 °C gelagert wurde.
(Fr. = Fraktion; Alc. anti-extr = ethanolischer Extrakt frischer Weizenkeime; Def alc anti-extr = ethanolischer Extrakt entfetteter Weizenkeime)

Die Untersuchungen zeigten zunächst, daß die antioxidative Wirkung des jeweiligen Gesamtextraktes frischer oder entfetteter Weizenkeime besser war als die Wirkung einer einzelnen Fraktion, vgl. die Figuren 27 - 30.

Im Vergleich der Extrakte frischer und entfetteter Weizenkeime erwiesen sich überraschenderweise die Extrakte frischer (d.h. fetthaltiger) Weizenkeime als wirksamer; dies ergibt sich aus der Gegenüberstellung der Figuren 27 und 29 sowie aus der Gegenüberstellung der Figuren 28 und 30.

Diese Ergebnisse sind möglicherweise auf einen synergistischen Effekt zwischen den verschiedenen Inhaltsstoffen des Roh-Extrakts frischer Weizenkeime zurückzuführen.

Desweiteren wurde festgestellt, daß die Unterschiede in der antioxidativen Wirkung zwischen den jeweils 6 Fraktionen (frisch oder fettfrei) beim Test unter Verwendung von tocopherolfreiem Maisöl sehr gering waren, vgl. die Fig. 27 und 29.

Bei dem Test unter Verwendung von Maisöl, das Tocopherol enthielt, zeigten die Fraktionen 5 (sowohl fetthaltig als auch entfettet) und 6 (entfettet) die höchsten antioxidativen Wirksamkeiten innerhalb der untersuchten Fraktionen, vgl. die Fig. 28 und 30. Die Unterschiede zwischen den Fraktionen waren (im Vergleich mit der Untersuchung tocoperolfreien Maisöls) allgemein erhöht, vgl. die Fig. 27 - 30.

### Beispiel 10: Vergleichstest zur Untersuchung des Einflusses der Rösttemperatur auf den antioxidativen Effekt extrahierter Weizenkeimextrakte bei der Behandlung von tocopherolfreiem Maiskeimöl

Analog zur Herstellung gerösteter Weizenkeime gemäß Beispiel 1 b) wurden frische Weizenkeime in einem 5-ml-Kolben mit Glasstopfen gefüllt und für 20 Minuten in einem Metallblock (Typ S-35-240, Firma Liebisch, Deutschland) bei einer Temperatur von
a) 140 °C,
b) 160 °C,
c) 180 °C bzw.
d) 200 °C
gehalten. Anschließend wurden die so bei unterschiedlichen Temperaturen gerösteten Weizenkeime jeweils mit flüssigem Stickstoff schockgekühlt.

Je 40 g der bei den unterschiedlichen Rösttemperaturen gemäß a) - d) erhaltenen gerösteten Weizenkeime wurden (analog Beispiel 2 c)) mehrmals kalt mit Ethanol extrahiert, so daß sich insgesamt vier Extrakte ergaben. Jeder dieser vier Extrakte wurde unter Verwendung eines Rotationsverdampfers bei 35 °C im Vakuum aufkonzentriert, sodaß das verbleibende Extrakt-Volumen 20 ml betrug. In 1 ml Extrakt waren somit die Inhaltsstoffe von 2 g gerösteten Weizenkeimen enthalten. Jeder der vier aufkonzentrierten Extrakte wurde quantitativ in einen 20 ml-Kolben überführt und zur weiteren Verwendung im Dunkeln bei -30°C gelagert.

Analog Beispiel 4 wurden dann 5 Proben zu je 50 g tocopherolfreies Maisöl ("stripped corn oil") untersucht. Diese Proben wurden in offene Becher mit einem Durchmesser von jeweils 8,6 cm gefüllt. Unter Rühren für 10 Minuten wurden dann vier der Proben mit je einem der vier ethanolischen Extrakte der bei unterschiedlichen Temperaturen gerösteten Weizenkeime versetzt (Dosierung 20 %, s. Anmerkung zu Beisp. 2). Die fünfte Probe wurde nicht mit einem antioxidativen ethanolischen Extrakt versetzt und diente Kontrollzwecken.

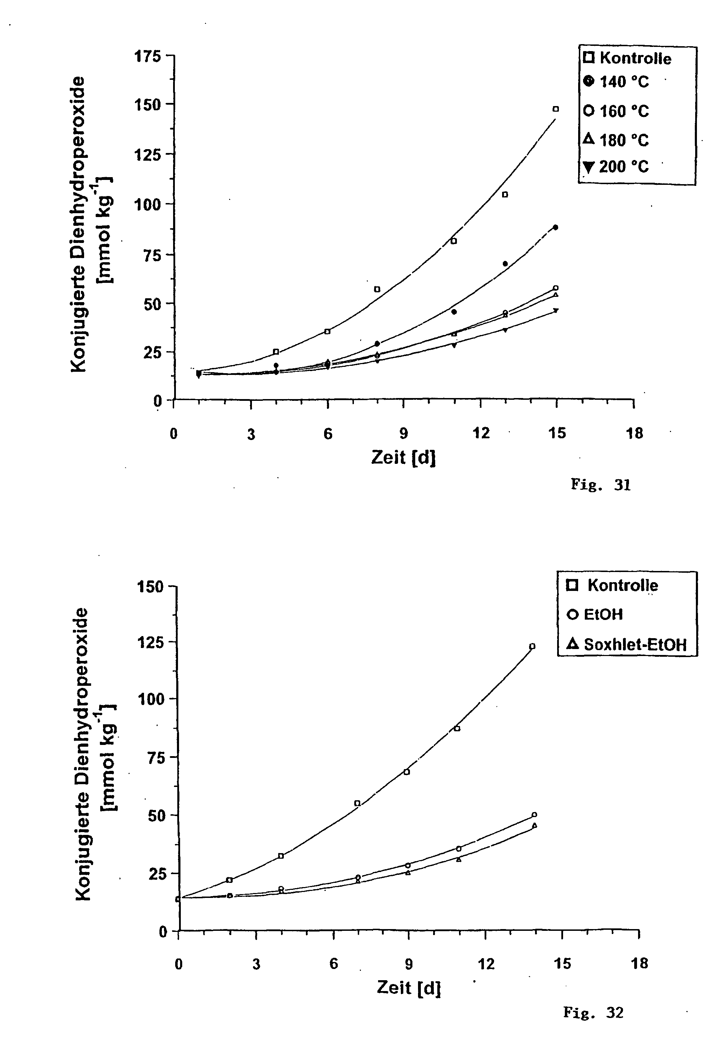

Die fünf Proben wurden bei 50°C im Trockenschrank gelagert. Die oxidative Stabilität der verschiedenen Proben wurde durch wiederholte Analyse ermittelt, wobei jeweils im Abstand mehrerer Tage (beginnend nach einem Tag) die Konzentration an konjugierten Dien-Hydroperoxiden bestimmt wurde, vergleiche Fig. 31.

Es stellt dar:
- Fig. 31: Konzentration an konjugierten Dien-Hydroperoxiden In tocopherolfreiem Maisöl, das bei 50°C mit unterschiedlichen ethanolischen Extrakten von Weizenkeimen gelagert wurde, die bei unterschiedlichen Temperaturen geröstet wurden.
(Kontrolle = Kontrollprobe; die den Meßpunkten zugeordneten Temperaturangaben in Fig. 31 indizieren die Rösttemperaturen der entsprechenden Proben)

Fig. 31 zeigt, daß die antioxidative Wirkung der ethanolischen Extrakte mit zunehmender Rösttemperatur der zugrundeliegenden Weizenkeime steigt. Bei Rösttemperaturen oberhalb von etwa 160 °C fallen die Verbesserungen gegenüber Extrakten aus Weizenkeimen, die bei geringerer Temperatur geröstet wurden, jedoch weniger stark aus. Eine Erhöhung der Rösttemperatur über die Temperatur von 160 °C hinaus bewirkt also nur noch geringe Verbesserungen hinsichtlich der antioxidativen Eigenschaften des entsprechenden Extraktes. Bei Rösttemperaturen von über 160-170 °C setzt stattdessen die Bildung toxikologisch bedenklicher Pyrolyseprodukte (IQ-Verbindungen, d.h. mutagene heterocyclische aromatische Amine) verstärkt ein; aus diesem Grunde sind höhere Rösttemperaturen nicht unbedingt erwünscht.

### Beispiel 11: Test zur Untersuchung des Einflusses des Extraktionsverfahrens auf die antioxidative Wirkung eines entsprechenden ethanolischen Extraktes.

Es wurden zwei auf unterschiedliche Weise hergestellte ethanolische Extrakte von gemäß Beispiel 1 b) gerösteten Weizenkeimen verglichen, nämlich:
(a) ein Extrakt gemäß Beispiel 2 c) und
(b) ein ethanolischer Soxhlet-Extrakt, der nach 20 Stunden kontinuierlicher Soxhlet-Extraktion von 40 g gerösteten Weizenkeimen gemäß Beispiel 1 b) mit Ethanol bei ca. zwei überläufen pro Stunde durch eine anschließende Aufkonzentration gemäß Beispiel 2 erhalten wurde.

Analog Beispiel 4 wurden dann 3 Proben zu je 50 g tocopherolfreies Maisöl ("stripped corn oil") untersucht. Diese Proben wurden in offene Becher mit einem Durchmesser von jeweils 8,6 cm gefüllt. Unter Rühren für 10 Minuten wurden dann zwei der Proben mit je einem der zwei auf unterschiedliche Weise erzeugten ethanolischen Extrakte versetzt (Dosierung 20 %, s. Anm. zu Beisp. 2). Die dritte Probe wurde nicht mit einem ethanolischen Extrakt versetzt und diente Kontrollzwecken.

Die Proben wurden bei 50°C im Trockenschrank gelagert. Die oxidative Stabilität der verschiedenen Proben wurde durch wiederholte Analyse ermittelt, wobei im Abstand mehrerer Tage jeweils die Konzentration an konjugierten Dien-Hydroperoxiden bestimmt wurde, vergleiche Fig. 32.

Es stellt dar:
- Fig. 32: Konzentration an konjugiertem Dien-Hydroperoxiden in tocopherolfreiem Maisöl, das bei 50°C mit auf unterschiedliche Weise erzeugten ethanolischen Extrakten gelagert wurde.
(Kontrolle = Kontrollprobe; EtOH = ethanolischer Extrakt gemäß Beispiel 2c; Soxhlet-EtOH = ethanolischer Soxhlet-Extrakt gemäß Beispiel 11b)

Aus Fig. 32 ergibt sich, daß eine kontinuierliche Extraktion gerösteter Weizenkeime (Kurve Soxhlet-EtOH) im Vergleich mit einer Extraktion gemäß Beispiel 2 c) (Kurve EtOH) zu keiner deutlichen Steigerung der antioxidativen Wirkung des resultierenden Extraktes führt.

### Beispiel 12: Test zur Untersuchung des Einflusses der Extraktmenge auf die antioxidative Wirkung eines ethanolischen Soxhlet-Extraktes

Eingesetzt wurde ein ethanolischer Soxhlet-Extrakt, der gemäß Beispiel 11 b) hergestellt worden war.

Analog Beispiel 4 wurden dann 5 Proben zu je 50 g tocopherolfreies Maisöl ("stripped corn oil") untersucht. Diese Proben wurden in offene Becher mit einem Durchmesser von jeweils 8,6 cm gefüllt. Unter Rühren für 10 Minuten wurden dann vier der Proben mit unterschiedlichen Mengen an Soxhlet-Extrakt versetzt, und zwar mit
(a) 5 % Soxhlet-Extrakt,
(b) 10 % Soxhlet-Extrakt,
(c) 20 % Soxhlet-Extrakt bzw.
(d) 40 % Soxhlet-Extrakt,
jeweils gemäß der Anmerkung zu Beispiel 2.

Die fünfte Probe wurde nicht mit einem ethanolischen Soxhlet-Extrakt versetzt und diente Kontrollzwecken.

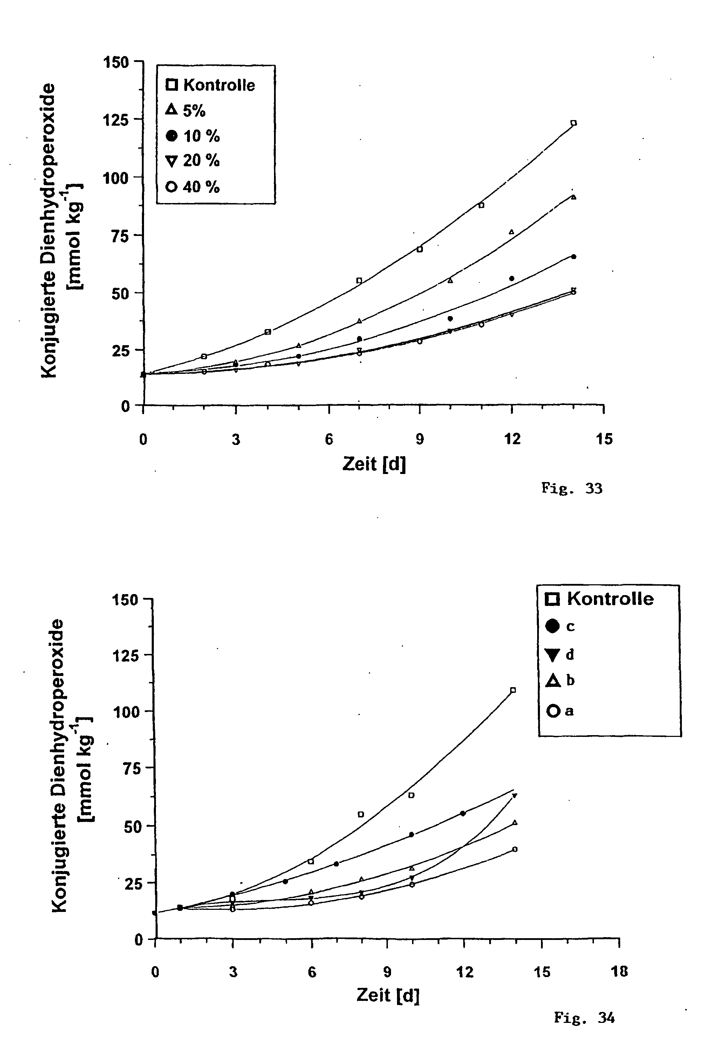

Die Proben wurden bei 50°C im Trockenschrank gelagert. Die oxidative Stabilität der verschiedenen Proben wurde durch wiederholte Analyse ermittelt, wobei im Abstand mehrerer Tage jeweils die Konzentration an konjugierten Dien-Hydroperoxiden bestimmt wurde, vergleiche Fig. 33.

Es stellt dar:
- Fig. 33: Konzentration an konjugierten Dien-Hydroperoxiden in tocopherolfreiem Maisöl, das bei 50 °C mit unterschiedlichen Mengen eines ethanolischen Soxhlet-Extraktes gelagert wurde.
(Kontrolle = Kontrollprobe; die den Meßpunkten zugeordneten Prozentangaben in Fig. 31 indizieren die unterschiedlichen eingesetzten Mengen an Extrakt)

Aus Fig. 33 ergibt sich, daß die Variation der Extraktmengen von 5 % auf 10 % eine deutlichen Verbesserung der antioxidativen Wirkung mit sich bringt.

Beim Übergang von 10 % auf 20% ist die Verbesserung der antioxidativen Wirkung nur noch gering.

Beim übergang von 20 % auf 40 % ist keine wesentliche Verbesserung der antioxidativen Wirkung mehr festzustellen.

### Beispiel 13: Vergleichender Test zur Stabilisierung tocopherolfreien Maisöls mit ethanolischen Extrakten unterschiedlicher gerösteter Getreidearten

### Untersucht wurden

a) ein ethanolischer Extrakt gemäß Beispiel 2 c), d.h. ein Extrakt, basierend auf den gerösteten Weizenkeimen gemäß Beispiel 1 b), sowie
b) ein ethanolischer Extrakt gerösteter Maiskeime, der analog Beispiel 2 c) aus gerösteten Maiskeimen hergestellt wurde, die analog zu Beispiel 1 b) geröstet worden waren,
c) ein ethanolischer Extrakt gerösteter Gerstenkeime, der analog Beispiel 2 c) aus gerösteten Gerstenkeimen hergestellt wurde, die analog zu Beispiel 1 b) geröstet worden waren,
Als weitere Vergleichssubstanz diente
d) Ascorbylpalmitat.

Es wurden vier Proben zu je 50 g tocopherolfreies Maisöl ("stripped corn oil") untersucht. Diese Proben wurden in offene Becher mit einem Durchmesser von jeweils 8,6 cm gefüllt. Unter Rühren für 10 Minuten wurden dann drei der Proben mit den vorstehend unter a) - c) beschriebenen Antioxidantien versetzt, d.h. mit Weizenkeimextrakt, Maiskeimextrakt und Gerstenkeimextrakt (Dosierung jeweils 20 %, vergleiche die Anm. zu Beispiel 2) bzw. Ascorbylpalmitat (Dosierung 0,02 Gew.%). Die fünfte Probe wurde nicht mit einem Antioxidans versetzt und diente Kontrollzwecken.

Die Proben wurden bei 50 °C gelagert. Die oxidative Stabilität der verschiedenen Proben wurde durch wiederholte Analyse ermittelt, wobei im Abstand mehrerer Tage (beginnend nach einem Tag) jeweils die Konzentration an konjugierten Dien-Hydroperoxiden bestimmt wurde, vgl. Fig. 34.

Es stellt dar:
- Fig. 34: Konzentration an konjugierten Dien-Hydroperoxiden in tocopherolfreiem Maisöl, das bei 50 °C mit unterschiedlichen Antioxidantien gelagert wurde.
(Kontrolle = Kontrollprobe; a = Weizenkeimextrakt; b = Maiskeimextrakt; c = Gerstenkeimextrakt; d = Ascorbylpalmitat)

Aus Fig. 34 ergibt sich, daß nicht nur der Weizenkeimextrakt, sondern auch der Maiskeimextrakt und der Gerstenkeimextrakt eine hervorragende antioxidative Wirkung besitzen.

Ascorbylpalmitat zeigte bei der Lagertemperatur von 50 °C eine antioxidative Wirkung, die für die ersten 10 Tage etwas schwächer war als die des Weizenkeimextraktes, jedoch stärker als die des Gersten- bzw. Maiskeimextraktes. Der rasche Fortschritt der Autoxidation vom 10. bis zum 14. Tag und eine Extrapolation der Kurve für Ascorbylpalmitat über den 14. Tag hinaus läßt aber eine prooxidative Wirkung zu einem späteren Zeitpunkt erwarten. Dies steht im Einklang mit der beobachteten pro-oxidativen Wirkung des Ascorbylpalmitats gemäß Beispiel 4.

Die untersuchten Extrakte von Weizen-, Gersten- und Maiskeimen lassen keine pro-oxidative Wirkung erwarten, auch nicht bei langen Lagerzeiten.

## Patentansprüche

1. Verfahren zur Herstellung eines antioxidativ wirksamen Extraktes, bei dem man von den sonstigen Bestandteilen eines Getreidekorns abgetrennte, nicht-enzymatisch gebräunte Getreidekeime mit einem einen E_{T}^{N}-Wert zwischen 0,6 und 0,8 besitzenden Lösungsmittel oder Lösungsmittelgemisch extrahiert und gegebenenfalls das Extraktionsmittel abtrennt.

2. Verfahren zur Herstellung eines antioxidativ wirksamen Extraktes, bei dem man geröstete, bereits vor dem Röstvorgang von den sonstigen Bestandteilen eines Getreidekorns abgetrennte Getreidekeime mit einem einen E_{T}^{N}-Wert zwischen 0,6 und 0,8 besitzenden Lösungsmittel oder Lösungsmittelgemisch extrahiert und gegebenenfalls das Extraktionsmittel abtrennt.

3. Verfahren nach Anspruch 1 oder 2, **dadurch gekennzeichnet, daß** als Extraktionsmittel Ethanol oder eine ethanolische Lösung verwendet wird.

4. Verfahren nach einem der vorangehenden Ansprüche, **dadurch gekennzeichnet, daß** als Getreidekeime Weizen-, Gersten- oder Maiskeime eingesetzt werden.

5. Verfahren nach einem der Ansprüche 2-4, **dadurch gekennzeichnet, daß** die Getreidekeime bei einer Rösttemperatur im Bereich zwischen 120 °C und 170 °C, vorzugsweise zwischen 140 °C und 160 °C, gebräunt werden.

6. Verfahren nach einem der Ansprüche 1-5, **dadurch gekennzeichnet, daß** man aroma- und/oder farbgebende Begleitstoffe des Extraktes von diesem abtrennt.

7. Antioxidativ wirksamer Extrakt zur Stabilisierung von Lebensmitteln, wobei der Extrakt nach einem Verfahren gemäß einem der Ansprüche 1 bis 6 herstellbar ist.

8. Verwendung einer Stoffmischung, die man durch Extraktion nichtenzymatisch gebräunter, von den sonstigen Bestandteilen eines Getreidekorns abgetrennter Getreidekeime gewinnt, oder einer Fraktion einer solchen Stoffmischung als antioxidativ wirksames Mittel zur Stabilisierung von Lebensmitteln, insbesondere lipidreichen Lebensmitteln, oder kosmetischen Mitteln.

9. Verwendung eines Extraktes nach Anspruch 7 oder einer Fraktion eines solchen Extraktes als antioxidativ wirksames Mittel zur Stabilisierung von Lebensmitteln, insbesondere lipidreichen Lebensmitteln, oder kosmetischen Mitteln.

10. Verfahren zur Stabilisierung von Lebensmitteln, insbesondere lipidreichen Lebensmitteln, oder kosmetischen Mitteln, in dem
- ein antioxidativ wirksamer Extrakt nach einem Verfahren gemäß einem der Ansprüche 1 bis 6 hergestellt wird
und
- das Lebensmittel oder kosmetische Mittel mit einer eine Stabilisierung bewirkenden Menge des Extrakts oder Fraktion des Extrakts behandelt wird.

11. Lebensmittel oder kosmetisches Mittel, umfassend eine stabilisierend wirkende Menge eines Extrakts nach Anspruch 7 oder einer Fraktion eines solchen Extraktes.

12. Verfahren zur Stabilisierung ungebräunter Getreidekeime, **dadurch gekennzeichnet, daß** man die ungebräunten Getreidekeime mit nichtenzymatisch gebräunten, von den sonstigen Bestandteilen eines Getreidekorns abgetrennten Getreidekeimen mischt.

13. Verfahren nach Anspruch 12, **dadurch gekennzeichnet, daß** ein Massen-Mischungsverhältnis von (a) nichtenzymatisch gebräunten, von den sonstigen Bestandteilen eines Getreidekorns abgetrennten zu (b) ungebräunten Getreidekeimen im Bereich von 2 : 100 bis 8 : 100, vorzugsweise im Bereich von 2 : 100 bis 4 : 100, eingestellt wird.

## Claims

1. Method of producing an extract having an antioxidant action, in which non-enzymatically browned cereal germs separated from the other constituents of a cereal grain are extracted with a solvent or a solvent mixture having an E_{T}^{M} value of between 0.6 and 0.8 and the extractant is separated off if required.

2. Method of producing an extract having an antioxidant action, in which roasted cereal germs which were separated from the other constituents of a cereal grain even before the roasting operation are extracted with a solvent or a solvent mixture having an E_{T}^{M} value of between 0.6 and 0.8 and the extractant is separated off if required.

3. Method according to claim 1 or 2, **characterised in that** ethanol or an ethanol solution is used as the extractant.

4. Method according to one of the foregoing claims, **characterised in that** wheat, barley or maize germs are used as the cereal germs.

5. Method according to one of claims 2-4, **characterised in that** the cereal germs are browned at a roasting temperature in the range between 120°C and 170°C, and preferably between 140°C and 160°C.

6. Method according to claims 1 - 5, **characterised in that** aromatising and/or colorant substances accompanying the extract are separated therefrom.

7. Extract having an antioxidant action for stabilising foodstuffs, where the extract can be produced by a method according to one of claims 1 to 6.

8. Use of a mixture of substances which is obtained by extracting non-enzymatically browned cereal germs which have been separated from the other constituents of a cereal grain, or of a fraction of such a mixture of substances, as a means having an antioxidant action for stabilising foodstuffs, and particularly high-lipid foodstuffs, or cosmetic media.

9. Use of an extract according to claim 7, or of a fraction of such an extract, as a means having an antioxidant action for stabilising foodstuffs, and particularly high-lipid foodstuffs, or cosmetic media.

10. Method of stabilising foodstuffs, and particularly high-lipid foodstuffs, or cosmetic media, in which
- an extract having an antioxidant action is produced by a method according to one of claims 1 to 6, and
- the foodstuff or cosmetic medium is treated with an amount of the extract or of the 'fraction of the extract which will produce stabilisation.

11. Foodstuff or cosmetic medium, comprising an amount having a stabilising action of an extract according to claim 7 or of a fraction of such an extract.

12. Method of stabilising unbrowned cereal germs, **characterised in that** the unbrowned cereal germs are mixed with non-enzymatically browned cereal germs which have been separated from the other constituents of a cereal grain.

13. Method according to claim 12, **characterised in that** a mixture ratio by weight of (a) non-enzymatically browned cereal germs which have been separated from the other constituents of a cereal grain to (b) unbrowned cereal germs is set in the range from 2:100 to 8:100 and preferably in the range from 2:100 to 4:100.

## Revendications

1. Procédé pour fabriquer un extrait à effet antioxydant, selon lequel on extrait des germes de céréale séparés des autres constituants d'un germe de céréale et brunis de façon non enzymatique avec un solvant ou un mélange de solvant présentant une valeur E_{T}^{N} comprise entre 0,6 et 0,8 et on sépare l'agent d'extraction éventuellement.

2. Procédé pour fabriquer un extrait à effet antioxydant, avec lequel on extrait des germes de céréale torréfiés et déjà séparés des autres constituants d'un grain de céréale avant l'opération de torréfaction avec un solvant ou un mélange de solvant présentant une valeur E_{T}^{N} comprise entre 0,6 et 0,8 et on sépare éventuellement l'agent d'extraction.

3. Procédé selon la revendication 1 ou 2, **caractérisé en ce qu'**on utilise comme agent d'extraction de l'éthanol ou une solution à base d'éthanol.

4. Procédé selon l'une quelconque des revendications précédentes, **caractérisé en ce qu'**on utilise comme germes de céréale des germes de blé, d'orge ou de maïs.

5. Procédé selon l'une quelconque des revendications 2 à 4, **caractérisé en ce que** les germes de céréale sont brunis à une température de torréfaction dans la plage comprise entre 120°C et 170°C, de préférence entre 140°C et 160°C.

6. Procédé selon l'une quelconque des revendications 1 à 5, **caractérisé en ce qu'**on sépare des substances associées donnant de l'arôme ou de la couleur de l'extrait de celui-ci.

7. Extrait à effet antioxydant pour la stabilisation de denrées alimentaires, l'extrait pouvant être fabriqué selon un procédé conformément à l'une des revendications 1 à 6.

8. Utilisation d'un mélange de substance, qu'on obtient par extraction de germes de céréale brunis de façon non enzymatique et séparés des autres constituants d'un grain de céréale, ou d'une fraction d'un tel mélange de substances comme produit à effet antioxydant pour la stabilisation de denrées alimentaires, en particulier de denrées alimentaires riches en lipides, ou de produits cosmétiques.

9. Utilisation d'un extrait selon la revendication 7 ou d'une fraction d'un tel extrait comme produit à effet antioxydant pour la stabilisation de denrées alimentaires, en particulier de denrées alimentaires riches en lipides ou de produits cosmétiques.

10. Procédé pour la stabilisation de denrées alimentaires, en particulier de denrées alimentaires riches en lipides, ou de produits cosmétiques, avec lequel
- un extrait à effet antioxydant est fabriqué selon un procédé conformément à l'une quelconque des revendications 1 à 6 et
- la denrée alimentaire ou le produit cosmétique est traité avec une quantité d'extrait ou une fraction d'extrait entraînant une stabilisation.

11. Denrée alimentaire ou produit cosmétique, comprenant une quantité d'un extrait selon la revendication 7 qui a un effet stabilisateur ou d'une fraction d'un tel extrait.

12. Procédé pour la stabilisation de germes de céréale non brunis, **caractérisé en ce qu'**on mélange les germes de céréale non brunis avec des germes de céréale brunis de. façon non enzymatique et séparés des autres composants d'un grain de céréale.

13. Procédé selon la revendication 12, **caractérisé en ce qu'**un rapport de mélange de masse entre des germes de céréale non brunis de façon enzymatique (a) et séparés des autres constituants d'un grain de céréale et des germes de céréale non brunis (b) est réglé dans la plage de 2/100 jusqu'à 8/100, de préférence dans la plage de 2/100 jusqu'à 4/100.
